Europäisches Patentamt

European Patent Office

Office européen des brevets

(11) Publication number: **0 362 821**
**A1**

# EUROPEAN PATENT APPLICATION

(21) Application number: **89118409.5**

(22) Date of filing: **04.10.89**

(51) Int. Cl.⁵: **A61B 6/00**

(30) Priority: **06.10.88 JP 252702/88**

(43) Date of publication of application:
**11.04.90 Bulletin 90/15**

(84) Designated Contracting States:
**DE NL**

(71) Applicant: **KABUSHIKI KAISHA TOSHIBA**
**72, Horikawa-cho Saiwai-ku**
**Kawasaki-shi Kanagawa-ken 210(JP)**

(72) Inventor: **Nishiki, Masayuki Intellectual**
**Property Division**
**KABUSHIKI KAISHA TOSHIBA 1-1 Shibaura**
**1-chome**
**Minato-ku Tokyo 105(JP)**
Inventor: **Honda, Michitaka Intellectual**
**Property Division**
**KABUSHIKI KAISHA TOSHIBA 1-1 Shibaura**
**1-chome**
**Minato-ku Tokyo 105(JP)**

(74) Representative: **Blumbach Weser Bergen**
**Kramer Zwirner Hoffmann Patentanwälte**
**Radeckestrasse 43**
**D-8000 München 60(DE)**

(54) Diagnostic X-ray apparatus.

(57) An X-ray emitted from an X-ray tube (12) and transmitted through an object (10) is imaged by an image intensifier (14) and a TV camera (18) to obtain an X-ray fluorogram. Angiography is performed prior to PTCA. An output from the TV camera (18) during angiography is written as road map images in an image memory (26). During PTCA, the X-ray fluorogram obtained from the TV camera (18) is supplied to a display (30) through a buffer memory (24). An output from the image memory (26) is supplied to a display (34). Read/write access of the image memory (26) is controlled by a read/write controller (38) which receives an output electrocardiogram from an electrocardiograph (36). A plurality of road map images are stored at equal time intervals within one heart beat period from a timing at which the electrocardiogram represents an R-wave and are read out in synchronism with the electrocardiogram. The road map images are read out at the same equal time intervals as those of the write mode. The road map image imaged when the electrocardiogram represents the R-wave is read out every time the electrocardiogram represents the R-wave. The subsequent road map images are read out, and the finally imaged road map image is repeatedly read out until the electrocardiogram represents the next R-wave.

FIG. 1

## Diagnostic X-ray apparatus

The present invention relates to a diagnostic X-ray apparatus for displaying a fluorogram of an object and a road map image for guiding a catheter to a target portion of the object.

Percutaneous transluminal coronary angioplastry (to be referred to as PTCA hereinafter) has been practiced to widen an inner cavity of a strictured coronary artery. In PTCA, a catheter having a balloon at its distal end is placed inside a strictured portion and the balloon is inflated to widen the strictured portion. In order to guide the catheter to the target portion, an X-ray fluorograpic apparatus is used. Prior to practicing of the PTCA, a contrast medium is injected into a blood vessel, angiography is performed, and a fluorogram (angiogram) showing blood vessels is obtained. One frame of this angiogram is stored in a memory as a road map image. During the practice of PTCA, the object is exposed with X-rays of a normal dose, and an X-ray fluorogram showing profiles of the respective organs of the object is obtained in real time on the basis of X-rays transmitted through the object. The road map image is displayed together with the fluorogram. The catheter inserted into the transluminal coronary of the object is operated with a guide wire and is moved to a desired target portion.

Although the fluorogram of the object is displayed in real time as a motion picture, the road map image as an X-ray fluorogram consisting of an image of blood vessels at a phase of heart beats is displayed as a single still image on the monitor. The phase of the road map image is not matched with that of the fluorogram which varies in real time. For this reason, even if a doctor observes the road map image, he cannot accurately judge the present position of the catheter at a timing except for the heart beat phase at which the road map image is stored. This problem also occurs in road map images in other treatments excluding the PTCA.

It is an object of the present invention to provide a simple diagnostic X-ray apparatus wherein a road map image can be synchronously displayed with an X-ray fluorogram with respect to a heart beat phase even if a heart beat period during photographing of the road map image is different from that during its display.

It is another object of the present invention to provide a display apparatus for displaying a road map image as a motion picture in synchronism with an actual fluorogram.

It is still another object of the present invention to provide a method of accurately displaying a road map image in synchronism with a heart beat phase during PTCA.

A diagnostic X-ray apparatus according to the present invention comprises: a TV camera for imaging an X-ray fluorogram of an object; an electrocardiograph for obtaining an electrocardiogram of the object; an image memory for storing a plurality of road map images; a read/write controller for writing in the image memory the plurality of X-ray fluorograms, as the road map images imaged at predetermined time intervals within one heart beat period from a timing at which the electrocardiogram represents an R-wave, the road map images representing blood vessels of the object, and for reading out the road map images in synchronism with the electrocardiogram from the image memory such that the road map images are sequentially read out at predetermined time intervals, a given road map image imaged when the electrocardiogram represents the R-wave is read out every time the electrocardiogram represents the R-wave, and a finally imaged road map image is repeatedly read out until the electrocardiogram represents the next R-wave: and a display for displaying the X-ray fluorogram imaged by the TV camera together with the road map images read out from the image memory.

The road map image display apparatus according to the present invention comprises a detector for detecting motion of a heart of an object, a unit for imaging road map images of the object at different phases of the motion of the heart of the object within one heart beat period and storing the imaged road map images, and a display for displaying the stored road map images in synchronism with the motion of the heart of the object.

A method of displaying a road map image according to the present invention comprises the steps of: imaging a fluorogram of an object to image an angiogram and obtaining an electrocardiogram; storing a plurality of angiograms as road map images at equal time intervals within any one period of the electrocardiogram prior to PTCA; and displaying the stored road map images in synchronism with a phase of the electrocardiogram.

This invention can be more fully understood from the following detailed description when taken in conjunction with the accompanying drawings, in which:

Fig. 1 is a block diagram of a diagnostic X-ray apparatus according to an embodiment of the present invention;

Fig. 2 is a view showing road map image fetch timings; and

Fig. 3 is a view showing road map image display timings.

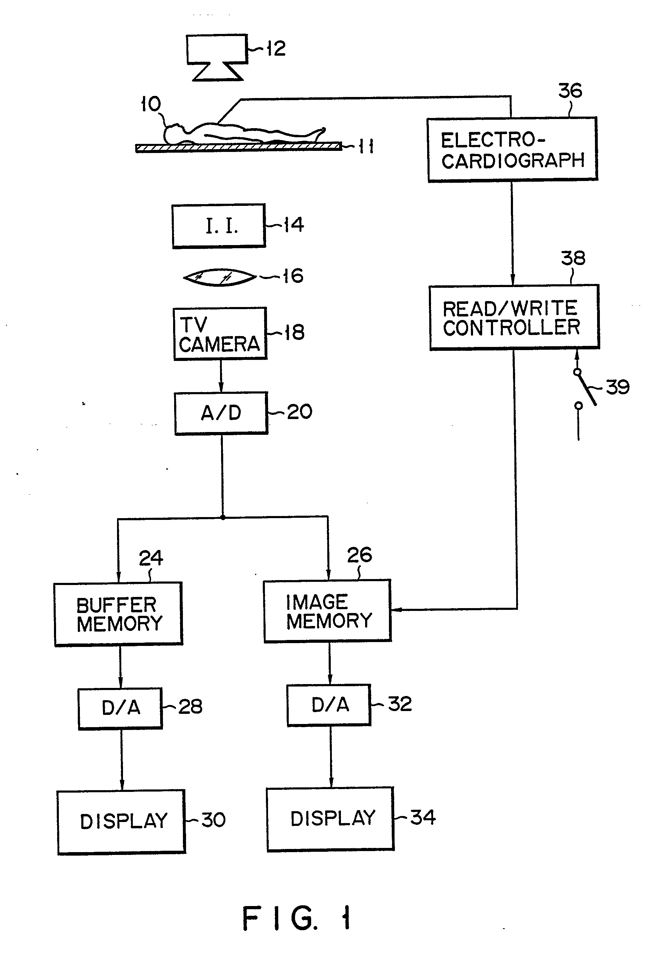

A diagnostic X-ray apparatus according to an embodiment of the present invention will be described with reference to the accompanying drawings. Fig. 1 is a block diagram showing an arrangement of the apparatus. An X-ray emitted from an X-ray tube 12 is transmitted through an object 10 on a bed 11. The transmitted X-ray is converted into an optical image by an image intensifier 14. The optical image represents a distribution of X-ray transmittances of the respective parts of the object. In this image, blood vessels cannot be visually distinguished from other organs. An optical image from the image intensifier 14 is incident on a TV camera 18 through an optical system 16 and is converted into an analog image signal. The X-ray tube 12, the object 10, the image intensifier 14, the optical system 16, and the TV camera 18 are arranged along the same line. The X-ray tube 12, the image intensifier 14, the optical system 16, and the TV camera 18 can be integrally rotated clockwise or counterclockwise around the body axis of object 10, and tilted. Therefore, an X-ray can be emitted onto the object 10 from any direction.

An output analog image signal from the TV camera 18 is converted into a digital image signal by an A/D converter 20. The digital image signal is supplied to either a buffer memory 24 or an image memory 26. The buffer memory 24 has a capacity for storing images of several frames and a FIFO function for sequentially outputting images (fluorograms representing profiles of the respective parts of the object) in an input order. An output from the buffer memory 24 is supplied to a display 30 through a D/A converter 28, and a fluorogram is displayed in real time as a motion picture on a display 30. On the other hand, the image memory 26 has a capacity for storing several tens of frames. The image memory 26 stores a plurality of road map images (e.g., angiograms) representing blood vessels within one heart beat period prior to PTCA and repeatedly outputs the stored images during PTCA. The output from the image memory 26 is supplied to a display 34 through a D/A converter 32 and the plurality of road map images are displayed on the display 34 as a motion picture. Read/write access of the image memory 26 is controlled by a read/write controller 38. The read/write controller 38 controls the read/write timing of the image memory 26 on the basis of an output (electrocardiogram) from an electrocardiograph 36 connected to the object 10 and a signal from an operation switch 39.

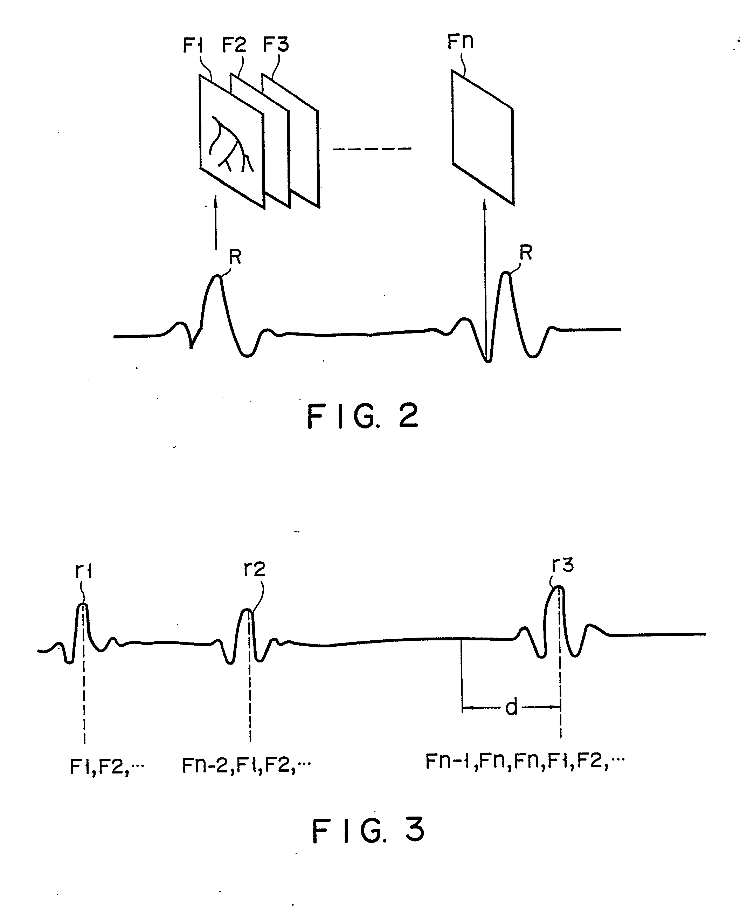

An operation of the above embodiment will now be described. Prior to PTCA, angiography is performed to obtain road map images. This operation need not be performed if an image representing blood vessels of the object can be obtained in fluorography. A contrast medium is injected into a blood vessel of the object to perform angiography, and the object 10 is exposed with an X-ray from the X-ray tube 12. The X-ray transmitted through the object 10 is converted into an optical image by the image intensifier 14. The optical image is converted into an analog image signal by the TV camera 18 through the optical system 16. The image signal is converted into a digital signal by the A/D converter 20. The digital signal is supplied to the display 30 through the buffer memory 24 and the D/A converter 28. An X-ray fluorogram is displayed in real time on the display 30. An output from the TV camera 18 need not be input to the display 30 through the A/D converter 20, the buffer memory 24, and the D/A converter 28, but can be directly input to the display 30. When an operator is observing the fluorogram and an angiogram serving as a road map image appears, the appearance of the angiogram is signalled to the controller 38 through the operation switch 39. Thereafter, the controller 38 monitors the electrocardiogram output from the electrocardiograph 36. After the controller 38 detects the first R-wave, a plurality of road map images (angiograms) output from the A/D converter 20 at predetermined time intervals within one heart beat period are written into the image memory 26. If an image write rate is defined as a frame rate of an NTSC television scheme, i.e., 30 frames/sec, and one heart beat period is one second, the number of images to be stored in the image memory 26 is 30. This operation is shown in Fig. 2. That is, road map images $F_1$ to $F_n$ ($n$ is an arbitrary positive integer, e.g., 30 in the above case) every 1/30 sec within one heart beat period from the R-wave of the object are stored in the image memory 26. More specifically, the controller 38 includes an address counter incremented at predetermined time intervals when the controller 38 detects the first R-wave of the electrocardiogram output from the electrocardiograph 36 after appearance of the angiogram is signalled. When the counter is incremented to $n$ in the write mode, its operation is stopped. In the read mode, the counter is incremented at the same predetermined time intervals as those in the write mode. It should be noted that the value $n$ is retained after the counter is incremented to $n$. The counter is reset to one upon appearance of the R-wave.

In the PTCA mode, the real-time fluorograms are displayed, while the road map images are read out from the image memory 26 and are displayed. In this case, the positional relationship between the object 10 and the X-ray tube 12 during imaging of the road map images is assumed to be the same as during PTCA. A digital image signal representing a fluorogram from the A/D converter 20 is converted into an analog signal by the D/A converter 28 through the buffer memory 24. The analog signal is displayed on the display 30 as a real-time fluorogram.

Meanwhile, the controller 38 reads out the first road map image $F_1$ from the image memory every time the R-wave of the electrocardiogram from the electrocardiograph 36 is detected. The road map images $F_1$, $F_2$,... are sequentially read out at the same frame rate as that of the write frame rate. The frame images $F_1$, $F_2$,... $F_n$ are sequentially displayed on the display 34 through the D/A converter 32. Thus, the road map images can be displayed as a motion picture. Since the read access of the frame images $F_1$, $F_2$,... are synchronized with the R-wave, the road map images synchronized with the fluorograms can be displayed, and accurate PTCA can be performed.

A heart beat period $T_1$ during imaging of the road map images is not necessarily matched with a heart beat period $T_2$ during PTCA. However, no problem is posed in this embodiment as will be described below. If condition $T_1 > T_2$ is established between an R-wave $r_1$ and an R-wave $r_2$ shown in Fig. 3, the next R-wave $r_2$ appears before all the n road map images are completely displayed. In this case, read access from the first road map image $F_1$ is repeated in synchronism with the R-wave $r_2$. In this manner, the display of the road map images obtained in a period toward the end of the expansion phase of the R-wave immediately before the next R-wave is omitted. During this period, the motion of the heart is very moderate, and no problem is posed. If condition $T_1 < T_2$ is established as between the R-wave $r_2$ and an R-wave $r_3$ shown in Fig. 3, the R-wave $r_3$ does not appear even after all the n road map images are completely displayed. In this case, the n-th road map image Fn is repeatedly displayed until the next R-wave $r_3$ is detected. An expansion phase end period d of the R-wave represents relatively moderate motion of the heart. Therefore, the n-th road map image $F_n$ may be repeatedly displayed.

According to this embodiment as has been described above, a plurality of road map images are stored in the image memory 26 at a predetermined frame rate in synchronism with the R-wave within one heart beat period of the electrocardiogram of the object. During PTCA, the road map images are read out in synchronism with the R-wave in an order of writing the images at the frame rate. The road map images synchronized with the fluorograms can be displayed as a motion picture. Therefore, the operator can accurately understand a catheter position in the object during PTCA. In addition, even if the heart beat period during storage of the road map images is different from that during PTCA, the road map image corresponding to the R-wave is synchronized with the R-wave of the electrocardiogram obtained during PTCA.

Therefore, the present invention can cope with variations in heart beat period with a simple arrangement.

As a modification of the present invention, a road map image may be superimposed on a fluorogram by combining the displays 30 and 34. The write access of the road map images in the image memory 26 is not limited to the one described above. For example, one heart beat period may be actually measured, and a write frame rate may be calculated in accordance with the capacity of the image memory 26 and the heart beat period. In this case, the read rate for display may be obtained by actually measuring one heart beat period, and the number of images stored in the image memory 26 and the measured heart beat period are used to calculate the read rate. In addition, the frame rate of the images written into the image memory 26 need not be fixed. Road map images may be written upon every detection of predetermined phases within one heart beat period. In this case, the phase of the electrocardiogram is detected even in the read mode, and a corresponding road map image is read out based on the detected phase. It should be noted that one heart beat period need not be one period from the R-wave.

## Claims

1. An X-ray apparatus comprising:
means for imaging an X-ray fluorogram of an object;
means for storing a road map image;
display means for displaying the X-ray fluorogram imaged by said imaging means together with the road map image read out from said storing means, characterized in that said road map image storing means comprises:
an electrocardiograph (38) for obtaining an electrocardiogram of the object;
means (26) for storing, as road map images, X-ray fluorograms representing blood vessels and imaged by said imaging means at predetermined time intervals within one heart beat period from a timing at which the electrocardiogram represents an R-wave; and
readout means (38) for reading out the road map images from said storing means in synchronism with the electrocardiogram such that the road map images are read out at the predetermined time intervals, a road map image obtained when the electrocardiogram represents the R-wave is read out every time the electrocardiogram represents the R-wave, subsequent road map images are sequentially read out in an imaging order, and a finally imaged road map image is repeatedly read out until the electrocardiogram represents the next R-wave.

2. An apparatus according to claim 1, characterized in that said display means comprises first display means (30) for displaying the X-ray fluorogram imaged by said imaging means and second display means (34) for displaying the road map images read out from said storing means.

3. An apparatus according to claim 1, characterized in that said display means (32, 34) comprises means for superimposing the X-ray fluorogram imaged by said imaging means and the road map images read out from said storing means.

4. An apparatus according to claim 1, characterized in that

said storing means (26) comprises means for storing the road map images in an imaging order at addresses which can be incremented sequentially from one; and

said readout means (38) comprises an address counter representing a read address of said storing means, said address counter being reset to one every time the electrocardiogram represents the R-wave, being incremented at the predetermined time intervals, and being maintained at a maximum value until resetting after the counter is incremented to the maximum value corresponding to the number of the road map images stored.

5. An apparatus according to claim 1, characterized in that said imaging means (12, 14, 18) comprises means for imaging an angiogram of a heart of the object, the angiogram being written in said storing means as the road map image.

6. A road map image display apparatus characterized by comprising:

means (36) for detecting motion of a heart of an object;

means for (12, 14, 18, 26) imaging and storing road map images representing blood vessels of the object at different phases of the motion of the heart of the object; and

readout means (38) for reading out the road map images in synchronism with the motion of the heart of the object.

7. An apparatus according to claim 6, characterized in that said imaging and storing means comprises means (26) for storing a plurality of angiograms imaged within one heart beat period from a specific phase.

8. An apparatus according to claim 6, characterized in that

said detecting means comprises an electrocardiograph (36);

said imaging and storing means comprises means (26) for storing a plurality of angiograms imaged at equal time intervals from a timing at which an electrocardiogram represents and R-wave; and

said readout means (38) comprises means for reading out the angiograms at said equal time intervals in an imaging order every time the electrocardiogram represents the R-wave.

9. A method of displaying a road map image in percutaneous transluminal coronary angioplastry (PTCA), characterized by comprising the steps of:

a) imaging an angiogram of an object and obtaining an electrocardiogram;

b) storing a plurality of angiograms as road map images at equal time intervals within any one period of an electrocardiogram prior to PTCA; and

c) imaging a fluorogram during PTCA and displaying the imaged fluorogram together with the stored road map images in synchronism with a phase of the electrocardiogram.

10. A method according to claim 9, characterized in that said b) storing step includes the step of storing a plurality of angiograms within one heart beat period from a timing at which the electrocardiogram represents an R-wave, and said c) imaging and displaying step includes the steps of reading out the angiograms at said equal time intervals, reading out the angiogram imaged at the timing at which the electrocardiogram represents the R-wave every time the electrocardiogram represents the R-wave, and repeatedly reading out a finally imaged angiogram until the electrocardiogram represents the next R-wave.

F I G. 1

F1  F2  F3                    Fn

R                              R

# FIG. 2

r1              r2                          r3

d

F1,F2,···    Fn-2,F1,F2,···       Fn-1,Fn,Fn,F1,F2,···

# FIG. 3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | DE-A-3 644 265 (K.K. TOSHIBA)<br>* Whole document *<br>--- | 1,3-8 | A 61 B 6/00 |
| A | US-A-4 585 008 (G.G. JARKEWICZ)<br>* Whole document *<br>--- | 1,4,6, 10 | |
| A | US-A-4 459 990 (D.I. BARNEA)<br>* Abstract; column 1, lines 15-30,61-66; column 2, lines 35-46; column 3, line 25 - column 4, line 50; figure 2 *<br>--- | 1,3,5-9 | |
| A | WO-A-8 300 970 (GEORGETOWN UNIVERSITY)<br>* Abstract *<br>----- | 1,2,5-7 | |

| | | | TECHNICAL FIELDS SEARCHED (Int. Cl.5) |
|---|---|---|---|
| | | | A 61 B |

The present search report has been drawn up for all claims

| Place of search | Date of completion of the search | Examiner |
|---|---|---|
| THE HAGUE | 13-12-1989 | FERRIGNO, A. |

EPO FORM 1503 03.82 (P0401)